# FASCICULE DE BREVET EUROPEEN

(11) **EP 0 814 853 B1**
(45) Date de publication et mention de la délivrance du brevet: **06.06.2001**
(21) Numéro de dépôt: 96908161.1
(22) Date de dépôt: 20.03.1996
(51) Int. Cl.: A61L 27/00

(54) **NOUVEAU MATERIAU PROTHETIQUE BIOCOMPATIBLE, PROTHESES OU SYSTEMES PROTHETIQUES CONSTITUES PAR LEDIT MATERIAU ET LEUR PROCEDE DE PREPARATION PAR DOUBLE IMPLANTATION IONIQUE DE CALCIUM ET DE PHOSPHORE**
BIOVERTRÄGLICHES PROTHESENMATERIAL, HERGESTELLTE PROTHESEN AUS DIESEM MATERIAL UND VERFAHREN ZU SEINER HERSTELLUNG DURCH EINE DOPPELTE IONENIMPLANTATION VON KALZIUM UND PHOSPHOR
NOVEL BIOCOMPATIBLE PROSTHETIC MATERIAL, PROSTHESES OR PROSTHETIC SYSTEMS CONSISTING OF SAID MATERIAL, AND METHOD FOR PREPARING SAME BY DUAL CALCIUM AND PHOSPHORUS ION IMPLANTATION

(30) Priorité: 20.03.1995 FR 9503198
(43) Date de publication de la demande: 07.01.1998
(73) Titulaire: Euros, 13600 La Ciotat (FR)
(72) Inventeur: TISSERAND, Philippe, F-66000 Perpignan (FR); SENEGAS, Jacques, F-33700 Mérignac (FR); VITAL, Jean-Marc, F-33200 Bordeaux (FR)
(74) Mandataire: CABINET BONNET-THIRION
(86) Numéro de dépôt international: FR9600421
(87) Numéro de publication internationale: WO9629101

(56) Documents cités:
- EP-A- 0 130 916
- EP-A- 0 548 365
- WO-A-88/08460
- BIOMATERIALS, vol. 13, no. 4, 1992, GB, pages 249-254, XP000006055 J.L. ONG ET AL.: "STRUCTURE, SOLUBILITY AND BOND STRENGTH OF THIN CALCIUM PHOSPHATE COATINGS PRODUCED BY ION BEAM SPUTTER DEPOSITION"

## Description

La présente invention concerne un nouveau matériau prothétique biocompatible, les prothèses ou systèmes prothétiques constitués par ledit matériau et leur procédé de préparation par double implantation ionique de calcium et de phosphore.

Les prothèses ou systèmes prothétiques employés en chirurgie doivent répondre à une double contrainte, d'une part mécanique et d'autre part biologique. En effet, les prothèses remplaçant des parties du squelette, elles s'y substituent en cherchant à imiter au mieux les propriétés mécaniques desdites parties remplacées, en particulier leur résistance mécanique. Les matériaux généralement employés pour des prothèses ou systèmes prothétiques sont donc de préférence des métaux ou alliages métalliques, en particulier constitués par du titane et ses alliages.

Toutefois, ces matériaux doivent également être biocompatibles, afin d'éviter les phénomènes de rejet, d'irritation, qui altèreraient le lien entre la prothèse et le tissu osseux dans lequel elle est implantée. Mieux, on cherchera à favoriser l'implantation des prothèses par des matériaux qui favorisent leur ancrage dans l'os auquel elles vont s'intégrer.

Diverses solutions ont été proposées dans l'état de la technique, dans lesquelles on formera à la surface du matériau prothétique, une couche destinée à assurer l'ancrage de la prothèse dans le tissu osseux. Il s'agit en général de couches phosphocalciques obtenues à partir d'hydroxyapatite, déposées à la surface du matériau prothétique par calcination et/ou au moyen d'une torche à plasma (EP-A-0 130 916, EP-A-0 264 354, EP-A-0 237 053, EP-A-0 264 353, EP-A-0 310 574, FR-A-2 601 699 ou FR-A-2 688 139).

Les prothèses ainsi obtenues sont donc constituées par un élément de prothèse métallique, ou en alliage métallique, assurant la résistance mécanique, recouvert, totalement ou en partie, par une couche d'un matériau phosphocalcique assurant la biocompatibilité, y compris l'ancrage, de la prothèse dans le tissu osseux. Toutefois, dans la mesure où ces prothèses sont constituées de deux matériaux distincts, il est nécessaire que la couche phosphocalcique, outre sa biocompatibilité, assure une bonne cohésion avec l'élément métallique, faute de quoi ce dernier ne pourrait assurer convenablement sa fonction de résistance mécanique. De la solidité de cette cohésion, c'est-à-dire sa résistance au décollement, dépendra l'efficacité et la longévité de la prothèse implantée.

La présente invention concerne donc un nouveau matériau prothétique amélioré, lequel ne nécessite pas d'être recouvert par une couche biocompatibilisante, et ne présente pas en conséquence de problèmes de décollement de ladite couche.

Le matériau prothétique selon l'invention est donc constitué par un matériau prothétique usuel, dont la surface a été traitée par une double implantation ionique de calcium et de phosphore, en quantités et en proportions suffisantes pour assurer sa biocompatibilté .

La technique de traitement de surface de matériaux par implantation ionique est connue depuis 1954, date à laquelle elle a été proposée pour doper les semi-conducteurs. Le principe de cette technique consiste à accélérer des ions positifs vers une cible portée à un potentiel négatif. Les ions ayant acquis une énergie cinétique suffisante, vont heurter et pénétrer la cible, c'est-à-dire s'intégrer au réseau atomique de la surface de cette dernière.

L'utilisation de l'implantation d'azote a été décrite pour le traitement de la surface de prothèses médicales (I. TOUSSET, L'implantation ionique, *Traitement Thermique,* 231, 1989, 11-16). Il s'agit d'implanter de l'azote, des gaz rares, du titane, du carbone, du silicium, du tantale ou encore du bore, à la surface des prothèses pour en améliorer la résistance à l'usure, à la corrosion, aux frottements. L'implantation d'azote à la surface de prothèses de hanches constituées d'un alliage de titane, permet ainsi d'améliorer leur résistance à l'usure corrosive, mais n'a pas d'incidence sur leur biocompatibilité, les solutions usuelles de l'état de la technique, reportées plus haut, étant nécessaires.

Par quantités suffisantes pour assurer la biocompatibilté, on entend une teneur en calcium et en phosphore à la surface du matériau selon l'invention suffisante pour assurer un bon ancrage dans la prothèse, sans que les propriétés physiques du matériau prothétique usuel ne soient pour autant altérées.

On a remarqué que les ions calcium, de par leur taille, étaient les plus difficiles à implanter à la surface des matériaux usuels. On cherchera donc de préférence à saturer la surface du matériau en calcium.

On ajustera ensuite la teneur en phosphore de manière à obtenir un rapport Ca/P approprié pour assurer la biocompatibilité du matériau prothétique selon l'invention.

De manière avantageuse, le rapport atomique Ca/P est compris entre 5/5 et 5/2, de préférence voisin de 5/3.

Le rapport Ca/P de 5/3 est celui que l'on retrouve dans le tissu osseux, et l'hydroxyapatite utilisée dans l'état de la technique, et on cherchera de préférence à implanter 5 ions calcium pour 3 ions phosphore.

Toutefois, la souplesse de la technique employée et son efficacité, permettra d'ajuster au mieux ce rapport Ca/P, en fonction du type de prothèse et du tissu osseux dans lequel elle sera implantée.

Par matériau prothétique usuel, on entend tout matériau servant à la préparation de prothèses osseuses et/ou dentaires, telles que des prothèses orthopédiques, articulaires ou des implants dentaires.

Il s'agit notamment de céramiques et de matériaux métalliques tels que le titane et ses alliages, les alliages chrome-cobalt ou encore l'acier inoxydable. Compte tenu de leur mode de préparation par implantation ionique, les matériaux employés peuvent être électriquement conducteurs ou non.

Le matériau actuellement le plus employé pour la réalisation de prothèses orthopédiques est un alliage de titane, appelé TA6V.

On a remarqué que le taux d'implantation maximum d'ions calcium à la surface d'un alliage de titane est d'environ 20 % en surface.

Bien entendu, la valeur de ce taux d'implantation ionique (de saturation) en calcium dépendra de la nature du matériau traité. Il sera généralement compris entre 20 et 25 %.

L'homme du métier saura toutefois adapter cette teneur en fonction du type de prothèse, du matériau usuel et de son implantation, et si nécessaire ne pas chercher uniquement à obtenir une saturation en calcium, mais toute teneur comprise entre la quantité minimum nécessaire à assurer la biocompatibilité du matériau et sa teneur maxium limitée par le type de matériau employé.

La présente invention concerne également les prothèses ou systèmes prothétiques constitués totalement ou en partie par le matériau biocompatible selon l'invention.

Ainsi, pour un système prothétique constitué de plusieurs éléments, comme par exemple un système d'implantation dentaire, seule la partie au contact du tissu osseux dans lequel le système sera implanté, sera constitué en matériau biocompatible selon l'invention.

Les prothèses ou systèmes prothétiques, dont la totalité de la surface sera au contact du tissu osseux, sont constitués entièrement par le matériau biocompatible selon l'invention.

La présente invention concerne également un procédé de préparation du matériau prothétique selon l'invention par implantation ionique, dans lequel on effectue d'abord l'implantation du calcium de manière à obtenir la teneur en calcium souhaitée, puis on effectue l'implantation du phosphore en ajustant le rapport atomique Ca/P recherché.

L'implantation sera faite dans un dispositif approprié pour implantation ionique, avec une énergie de préférence de 300 keV.

Pour la réalisation des prothèses et/ou systèmes prothétiques selon l'invention, on effectuera d'abord une mise en forme du matériau prothétique usuel à traiter, puis on effectuera une double implantation ionique telle que définie ci-dessus, et le cas échéant on assemblera le matériau ainsi obtenu avec d'autres éléments de prothèses ou de systèmes prothétiques.

De tels éléments additionnels peuvent être par exemple des éléments de prothèse dentaire coopérant avec le matériau implanté dans l'os, ou encore un dispositif facilitant le frottement de divers éléments dans un système de prothèse articulaire.

D'autres caractéristiques du matériau et du procédé selon l'invention apparaîtront à la lumière des exemples ci-après.

### 1. IMPLANTATION IONIQUE SUR UN MATÉRIAU EN ALLIAGE TA6V

Une pièce en alliage de titane TA6V a été implantée en surface, d'abord en calcium, puis en phosphore, en accélérant les ions correspondants grâce à une énergie de 300 KeV.

Cette implantation se fait de façon à ce que le rapport Ca/P soit égal à 1,667 (5 ions de Ca pour 3 ions de P), sur 25 % de la surface totale de la pièce en alliage.

### 2. ETUDE DE BIOCOMPATIBILITÉ

### Matériels et méthodes

Les disques d'alliages implantés obtenus ci-dessus ont été mis dans des boîtes de culture NUNC 4 puits de diamètre 2 cm dans une salle de type P2. Le témoin sera le polystyrène (PS) du fond des puits de culture. Ce témoin permet une prolifération optimum des ostéoblastes.

Les boîtes ont été ensuite ensemencées avec des ostéoblastes humains. Les cellules suspendues dans du milieu IMDM (Iscoves Modified Dubbelco Medium) supplémenté à 20 % de SVF (Sérum de Veau Foetal) et 100 U/ml de streptomycine et de pénicilline ainsi que 100 mg/ml de fungizone ont été ensemencées à 3 000 cellules/cm² de matériau. Les boîtes ont ensuite été incubées en atmosphère humide à 5 % de CO₂. Le milieu de culture a été changé tous les deux jours jusqu'à confluence.

Décollées avec un mélange Trypsine / EDTA (0,01 % de Trypsine et 0,02 % EDTA), les cellules une fois resuspendues ont été comptées dans une cellule de Malassez.

### Changement de milieu

Le milieu introduit le premier jour permet de donner aux cellules les éléments nutritifs nécessaires à leur croissance et à leur prolifération. Ainsi, elles utilisent ces éléments qui disparaissent alors de la solution. Ce milieu est donc changé tous les deux jours.

Le changement de milieu doit se faire en atmosphère stérile, le protocole expérimental a été le suivant :
. l'ancien milieu a été aspiré,
. le nouveau milieu complet a été introduit à raison de 500 µl par puits.

### Comptage cellulaire

L'évolution de la prolifération est suivie sur les différents matériaux. Pour cela, les ostéoblastes présents sur chaque type d'échantillon sont comptés. Ici le comptage a été fait sur trois échantillons différents. Le protocole expérimental a été le suivant :
. aspiration du milieu,
. rinçage trois fois dix minutes au PBS (Phosphate Buffered Saline),
. décollement du tapis cellulaire par 300 µl d'un mélange trypsine-EDTA, le mélange est mis à incuber avec les cellules à 37°C pendant 3 minutes. Le rôle de ce mélange est de digérer la matrice.
. 200 µl de milieu complet ont été ajoutés pour compléter à 500 µl,
. après avoir été homogénéisée, une petite quantité de la solution a été aspirée avec une pipette Pasteur et a été mise dans une cellule de Malassez.

### Résultats

Cette expérience a permis de tester la cytocompatibilité du TA6V ayant subi une double implantation de calcium et de phosphore (I). Ceci a été fait avec des ostéoblastes. Un premier témoin négatif TA6V non implanté (T) a été utilisé ainsi que le témoin négatif habituel, à savoir le polystyère (PS) du fond des puits de culture.

L'ensemencement s'est fait à 3 000 cellules/cm².

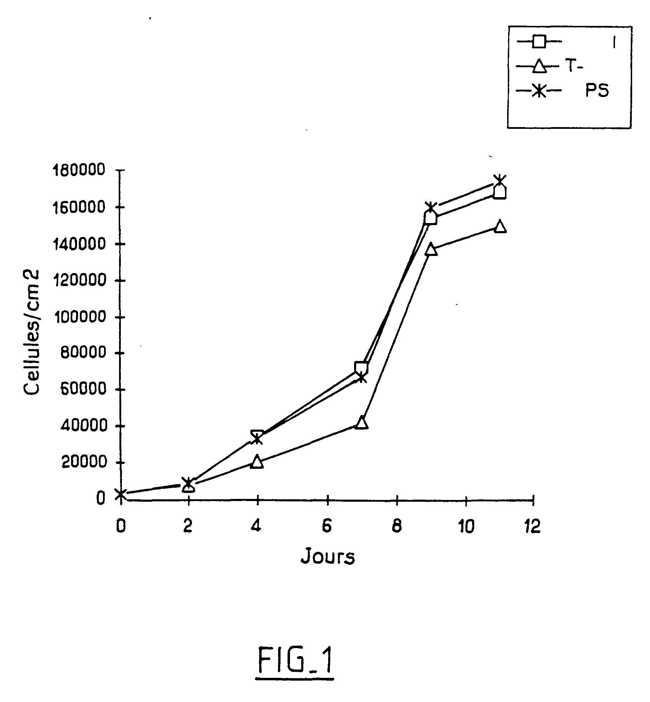

La prolifération d'ostéoblastes sur du TA6V implanté (I) et sur les témoins (T et PS) est illustrée sur la figure 1 en annexe. Un retard de prolifération des cellules a été observé pour le témoin TA6V sans traitement (T) par rapport à l'échantillon TA6V implanté (I). Par contre, sur le TA6V implanté (I) la prolifération cellulaire a été identique à celle du témoin polystyrène (PS) (surface de référence pour les cellules) à toutes les étapes du comptage (pas de différence statistiquement significative p < 0.05). Un plateau dans la prolifération a pu être observé à partir du 9ème jour de croissance pour tous les échantillons. Il a pu être observé une croissance exponentielle des cellules jusqu'au 9ème jour pour le témoin PS et pour le TA6V implanté (I). On peut voir dans ce cas que l'implantation ionique de calcium et de phosphore a permis d'améliorer la biocompatibilité du TA6V puisque la prolifération cellulaire sur le TA6V implanté (I) est identique à celle sur le témoin PS.

Ainsi, malgré le fait que seulement 25 % de la surface totale de l'alliage TA6V soit implantée en calcium et en phosphore, on obtient une prolifération identique à celle du polystyrène, témoin négatif habituel du fond des puits de culture.

## Revendications

1. Matériau prothétique biocompabile, caractérisé en ce qu'il est constitué par un matériau prothétique usuel, dont la surface a été traitée par une double implantation ionique de calcium et de phosphore, en quantités et en proportions suffisantes pour assurer sa biocompatibilité.

2. Matériau prothétique selon la revendication 1, caractérisé en ce que la surface du matériau prothétique usuel est saturée en calcium.

3. Matériau prothétique selon la revendication 2, caractérisé en ce que le taux de calcium à la surface du matériau prothétique usuel est compris entre 20 et 25 % en surface.

4. Matériau prothétique selon l'une des revendications 1 à 3, caractérisé en ce que le rapport atomique Ca/P est compris entre 5/5 et 5/2, de préférence voisin de 5/3.

5. Matériau prothétique selon l'une des revendications 1 à 4, caractérisé en ce que le matériau prothétique usuel est choisi parmi les céramiques, les métaux et les alliages métalliques, de préférence le titane et ses alliages.

6. Prothèses ou systèmes prothétiques, caractérisés en ce qu'ils sont constitués, totalement ou en partie, par le matériau prothétique selon l'une des revendications 1 à 5.

7. Procédé de préparation du matériau prothétique selon l'une des revendications 1 à 6, caractérisé en ce que l'on effectue d'abord l'implantation du calcium de manière à obtenir la teneur en calcium souhaitée, puis on effectue l'implantation du phosphore en ajustant le rapport atomique Ca/P recherché.

8. Procédé selon la revendication 7, caractérisé en ce que l'on effectue l'implantation ionique dans un dispositif approprié, avec une énergie de 300 KeV.

9. Procédé de préparation d'une prothèse ou d'un système prothétique selon la revendication 6, caractérisé en ce que l'on effectue d'abord une double implantation ionique telle que définie dans l'une des revendications 7 ou 8, et le cas échéant on assemble le matériau ainsi obtenu avec d'autres éléments de prothèse ou de système prothétique.

## Patentansprüche

1. Bioverträgliches Prothesenmaterial, dadurch gekennzeichnet, dass es aus einem gebräuchlichen Prothesenmaterial besteht, dessen Oberfläche durch eine doppelte Ionenimplantation von Calcium und Phosphor in ausreichenden Mengen und Verhältnissen, um seine Bioverträglichkeit zu gewährleisten, behandelt wurde.

2. Prothesenmaterial nach Anspruch 1, dadurch gekennzeichnet, dass die Oberfläche des gebräuchlichen Prothesenmaterials mit Calcium gesättigt ist.

3. Prothesenmaterial nach Anspruch 2, dadurch gekennzeichnet, dass der Calciumgehalt an der Oberfläche des gebräuchlichen Prothesenmaterials 20 bis 25 % der Oberfläche beträgt.

4. Prothesenmaterial nach einem der Ansprüche 1 bis 3, dadurch gekennzeichnet, dass das Atomverhältnis Ca/P zwischen 5/5 und 5/2 und vorzugsweise nahe bei 5/3 liegt.

5. Prothesenmaterial nach einem der Ansprüche 1 bis 4, dadurch gekennzeichnet, dass das gebräuchliche Prothesenmaterial aus Keramiken, Metallen und Metalllegierungen, vorzugsweise Titan und seinen Legierungen ausgewählt ist.

6. Prothesen oder Prothesensysteme, dadurch gekennzeichnet, dass sie ganz oder teilweise aus dem Prothesenmaterial nach einem der Ansprüche 1 bis 5 bestehen.

7. Verfahren zur Herstellung des Prothesenmaterials nach einem der Ansprüche 1 bis 6, dadurch gekennzeichnet, dass man zunächst die Implantation von Calcium so vornimmt, dass man den gewünschten Calciumgehalt erhält, und dann die Implantation des Phosphors vornimmt, indem man das gewünschte Verhältnis Ca/P einstellt.

8. Verfahren nach Anspruch 7, dadurch gekennzeichnet, dass man die Ionenimplantation in einer geeigneten Vorrichtung mit einer Energie von 300 KeV vornimmt.

9. Verfahren zur Herstellung einer Prothese oder eines Prothesensystems nach Anspruch 6, dadurch gekennzeichnet, dass man zunächst eine doppelte Ionenimplantation nach einem der Ansprüche 7 oder 8 vornimmt und gegebenenfalls das auf diese Weise erhaltene Material mit anderen Protheseelementen oder Prothesesystemen verbindet.

## Claims

1. A biocompatible prosthetic material characterised in that it is formed by a customary prosthetic material, the surface of which has been treated by double ionic implantation of calcium and phosphorus, in amounts and proportions which are sufficient to ensure its biocompatibility.

2. A prosthetic material according to claim 1 characterised in that the surface of the customary prosthetic material is calcium-saturated.

3. A prosthetic material according to claim 2 characterised in that the proportion of calcium at the surface of the customary prosthetic material is between 20 and 25% in relation to surface area.

4. A prosthetic material according to one of claims 1 to 3 characterised in that the Ca/P atomic ratio is between 5/5 and 5/2, preferably close to 5/3.

5. A prosthetic material according to one of claims 1 to 4 characterised in that the customary prosthetic material is selected from ceramics, metals and metal alloys, preferably titanium and alloys thereof.

6. Prostheses or prosthetic systems characterised in that they are entirely or partially formed by the prosthetic material according to one of claims 1 to 5.

7. A process for preparation of the prosthetic material according to one of claims 1 to 6 characterised by firstly effecting implantation of the calcium so as to obtain the desired calcium content, then effecting implantation of the phosphorus while adjusting the Ca/P atomic ratio sought.

8. A process according to claim 7 characterised by effecting the ionic implantation operation in a suitable apparatus with an energy of 300 KeV.

9. A process for preparation of a prosthesis or a prosthetic system according to claim 6 characterised by first effecting double ionic implantation as defined in one of claims 7 and 8 and if necessary assembling the material obtained in that way with other prosthesis or prosthetic system elements.
